# EUROPEAN PATENT APPLICATION

(11) **EP 4 378 398 A2**
(43) Date of publication of application: **05.06.2024**
(21) Application number: 24164684.3
(22) Date of filing: 12.04.2019
(51) Int. Cl.: A61B 17/00

(54) **STEERABLE ULTRASOUND ATTACHMENT FOR ENDOSCOPE**

(30) Priority: 12.04.2018 US 201815951347
(62) Divisional of application: 19721925.6
(71) Applicant: ENDOSOUND, INC., Portland, OR 97239 (US)
(72) Inventor: STEINBERG, Stephen E., Boca Raton, 33432 (US); CORBETT, Scott Sutherland III, Portland, 97219 (US)
(74) Representative: Snipe, Benjamin Thomas Fletcher

(57) **Abstract**

An ultrasonic endoscope assembly (110), having an endoscope (111) defining one or more lumens, and a needle (114) that can be pushed forward out of a lumen, to collect a biopsy specimen. An ultrasound sub-assembly (120) is attached to the endoscope (111) and includes a communications cable (126) including a set of conductors, having a distal end and an ultrasound imaging head (122) connected to the distal end of the cable. Also, included is an imaging head movement sub-assembly (140), including a conduit (148) that is releasably connected to the endoscope. The conduit contains a tension member (144) that is releasably connected to the imaging head (122). Accordingly, the imaging head movement sub-assembly (140) can be released from the cable and the imaging head, to be disposed of, after use.

## Description

### Background

### 1. Field of the Invention

The Invention is in the field of ultrasound imaging add-on equipment for endoscopes.

### 2. Background Art

Endoscopic ultrasound has undergone a rapid pace of development, now being used for the diagnosis and treatment of a wide variety of medical problems. As an endoscope can reach a location in the intestinal tract, closer than any skin surface, there is an opportunity to image from a closer location, and to obtain a tissue sample, using a biopsy needle and implement a variety of treatments. But due to an expense of greater than $200,000 for a complete system, endoscopic ultrasound systems are generally restricted to major hospitals. Endoscopes, however, are used in physicians' offices, most outpatient surgery centers and virtually all hospitals.

One type of endoscope is an upper endoscope, used to image and take tissue specimens from the upper GI tract. In this type of endoscope, if a needle is used to collect a specimen, it is typically advanced straight out of an endoscope lumen in a distal direction. Other types of endoscopes are bronchoscopes for viewing air passageways in the lungs and colonoscopes for viewing the colon.

Yet another type of endoscope is a duodenoscope, designed to be introduced into the duodenum (the upper part of the small intestines), and typically used to perform endoscope retrograde cholangiopancreatography (ERCP), in which the pancreas is imaged. Duodenoscopes are also used to gather tissue biopsies from sites in the duodenum, including the bile ducts. Duodenoscopes typically have a tip that houses a light, a video camera, a needle and a needle guide that can be tilted by an operator to control the angle at which the needle advances. Although the video camera and light can produce imagery that may help a surgeon guide the needle to a good biopsy site, ultrasound imagery, when available, can prove a valuable additional source of information. An ultrasound add-on for endoscopes has been described, but its capabilities are limited in that the viewing angle of the imaging head cannot be adjusted.

A problem faced by practitioners in the field of endoscopy is the thorough disinfection of the endoscope, between uses. As many endoscopes, in particular duodenoscopes, have some mechanical complexity, introducing a sterilizing material into the small spaces defined by these mechanisms, creates a huge challenge.
Recently, an endoscope mechanism, having a needle angle adjustment mechanism that is removable and disposable has been introduced, addressing many of these issues.

### Brief Description of the Drawings

Exemplary embodiments are illustrated in referenced drawings. It is intended that the embodiments and figures disclosed herein are to be considered illustrative rather than restrictive.
FIG. 1 is an isometric view of an imaging assembly having an endoscope and an ultrasound imaging assembly added on, according to a preferred embodiment of the present invention.
FIG. 2 is an isometric view of the distal end of the assembly of FIG. 1.
FIG. 3 is a side view of the distal end of the assembly of FIG. 1, in a first position.
FIG. 4 is a side view of the distal end of the assembly of FIG. 1, in a second position.
FIG. 5 is a side view of the distal end of an alternative embodiment of an imaging assembly.
FIG. 6 is a side view of another alternative embodiment of an imaging assembly.
FIG. 7 is an alternative embodiment of an imaging assembly, having an ultrasound imaging sub-assembly that includes a ultrasound head movement assembly that is detachable, and showing three alternative needle guides.
FIG. 8 is a sectional view of a of the assembly of FIG. 7, showing a needle guide in a deployed state.
FIG. 9 is a detail view of a portion of the assembly of FIG. 7, showing two of the parts disassembled from each other.
FIG. 10 is the detail view of FIG. 9 but showing the two parts joined. FIG. 11 is a sectional view taken along line 11-11, of FIG. 10.
FIG. 12 is a sectional view taken along line 12-12 of FIG. 10.
FIG. 13 is a sectional view of the tip of the assembly of FIG. 7, showing the needle guide used to guide a needle.
FIG. 14 is a sectional partial view of the assembly of 7 having a different style of needle guide.
FIG. 15 is a view of the assembly of Figure 14, showing the needle guide in use.
FIG. 16 is an isometric view of a duodenoscope assembly, having an ultrasound imaging sub-assembly.
FIG. 17 is an isometric view of the assembly of FIG. 16, in a disassembled state.
FIG. 18 is a sectional view of the assembly of FIG. 16, showing a different position for a portion of the assembly, in dashed line.
FIG. 19 is an isometric view of an alternative embodiment of a duodenoscope assembly.
FIG. 20 is an isometric of the assembly of FIG. 19, in a disassembled state.

### Summary

In a first separate aspect, the present invention may take the form of a method of adding elements to an endoscope, to create an ultrasound capable endoscope. The method utilizes an ultrasound assembly that includes a multiconductor electrical connector and a deformable neck, having a proximal end and a distal end. An ultrasound transducer head is supported by the distal end of the neck and includes an ultrasound transducer. Also, a tension member is connected to the ultrasound transducer head and extends to the proximal end of the endoscope. Finally, a set of signal pathways extend from the ultrasound transducer to the multiconductor electrical connector. In the method the ultrasound assembly is attached to the endoscope, so that the ultrasound assembly extends along the elongate body of the endoscope, and so that the ultrasound transducer head is held by the neck at a position distal to the distal end of the endoscope, and the tension member is arranged so that it extends from a proximal point of the endoscope to the transducer head. After the elements are added to the endoscope, the ultrasound transducer head can be introduced into a patient body cavity, with the endoscope, and may be angle adjusted by the tension member by pulling on the tension member to bend the neck thereby adjusting position of the ultrasound transducer head, and may communicate with an imaging station by means of the signal pathways, thereby permitting a user to more accurately aim the imaging head at a feature of interest. Finally, the neck defines an aperture, permitting a needle to be extended from a lumen of the endoscope, through the aperture, to perform a medical procedure, when the neck is flexed by the tension member.

In a second separate aspect, the present invention may take the form of an ultrasonic endoscope assembly, having an endoscope defining one or more lumens, and a needle that can be pushed forward out of a lumen, to collect a biopsy specimen. An ultrasound sub-assembly is attached to the endoscope and includes a communications cable, including a set of signal pathways, and having a distal end and an ultrasound imaging head connected to the distal end of the cable. Also, included is an imaging head movement sub-assembly, including a conduit that is releasably connected to the endoscope. The conduit contains a tension member that is releasably connected to the imaging head. Accordingly, the imaging head movement sub-assembly can be released from the endoscope and the imaging head, to be disposed of, after use.

In a third separate aspect, the present invention may take the form of a method of processing endoscope assemblies, between use, wherein each endoscope assembly includes an endoscope, an ultrasound sub-assembly, including a communications cable connected to an ultrasound imaging head, and an imaging head movement sub-assembly. After surgical use, removing and disposing of the imaging head movement sub-assembly and removing and cleaning the ultrasound sub-assembly and cleaning the endoscope, thereby creating a cleaned endoscope. Before use, taking a cleaned ultrasound sub-assembly and placing it on the cleaned endoscope. Also, opening a clean package containing a never-used imaging head movement sub-assembly and attaching it to the endoscope and ultrasound sub-assembly, thereby joining the endoscope, ultrasound sub-assembly and imaging head movement sub-assembly together.

In a fourth separate aspect, the present invention may take the form of an endoscope add-on assembly adapted to be attached to a target endoscope. The assembly includes an ultrasound imaging sub-assembly, including a communications cable connected to an ultrasound imaging head and an imaging head movement sub-assembly, including a conduit, holding a tension member that is attached to the ultrasound imaging head. Further included are connective elements, adapted to permit the endoscope add-on assembly to be attached to the target endoscope. Finally, the imaging head movement sub-assembly is detachable from the ultrasound imaging sub-assembly, thereby permitting the imaging head movement subassembly to be processed separately from the ultrasound imaging sub-assembly, after use.

In a fifth separate aspect, the present invention may take the form of a method of adjusting the viewing angle of an imaging assembly having an endoscope and an added ultrasound imaging array. The array is part of an ultrasound assembly that includes a multiple signal pathway connector; a multiple signal pathway cable having a distal end, an endoscope attachment element, attached to the multiple signal pathway cable, and also attached to the distal end of the endoscope; a neck, connected and extending in a distal manner from the distal end of the multiple signal pathway cable, and having a distal end, the neck being resiliently deformable; an ultrasound transducer head, including a protective covering, supported by the distal end of the neck, and an ultrasound transducer, inside the protective covering and electrically connected to the distal end of the multiple signal pathway cable; and a tension member, connected to the ultrasound transducer head, and extending to the proximal end of the endoscope so that a free end of the tension member is accessible to a human operator. The method begins, once the multiple signal pathway connector has been connected to an ultrasound imaging station and the ultrasound transducer head has been introduced into a cavity of a patient, with the endoscope and includes pulling on the free end the tension member to cause the ultrasound transducer head to move from a position that is aligned to the distal end of the endoscope to a position bent at an obtuse angle relative to the distal end of the endoscope and permitting the free end to retract toward the lumen, thereby permitting the ultrasound transducer head to move back toward the position aligned to the distal end of the endoscope, thereby scanning a portion of the patient's internal organs.

### Best Modes of Carrying out the Invention

### Definition

As used in this application, the term "endoscope" refers to an illuminated optical, typically slender and tubular instrument used to look deep into the body and used in procedures referred to as "endoscopy". This term encompasses, but is not limited to upper endoscopes, duodenoscopes, colonoscopes and bronchoscopes, as well as devices referenced simply as "endoscopes".

### Description

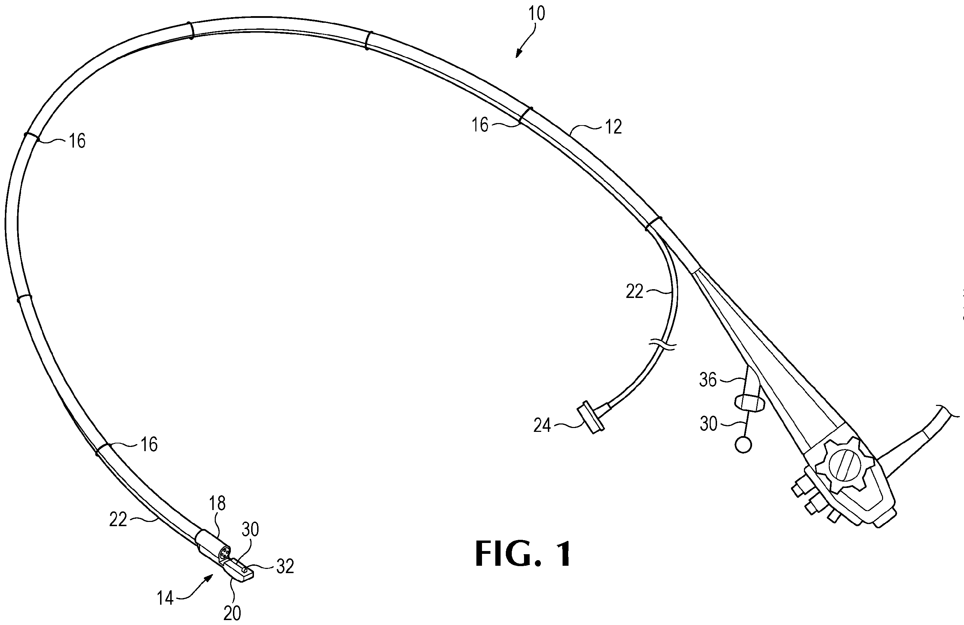

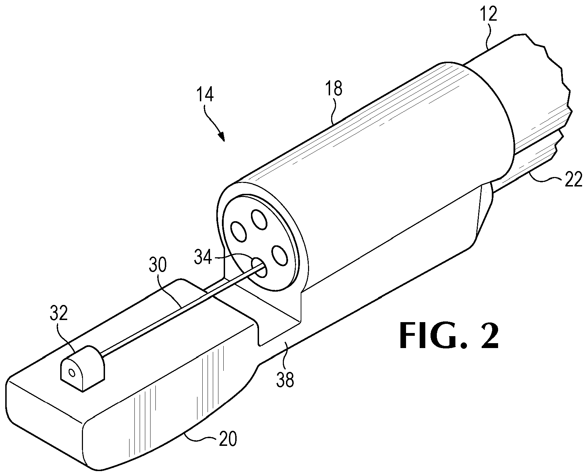

In a first preferred embodiment, an imaging assembly 10 includes an upper endoscope 12 and an ultrasound assembly 14 that has been attached to endoscope 12 by means of retaining element 18, integral to ultrasound assembly 14. Assembly 14 also includes an ultrasound imaging (also referred to as "transducer") head 20 that is electrically connected to a multiple signal pathway cable 22 by way of a flex circuit 50 (which is also a form of a signal pathway cable), that includes a set of parallel electrical leads, which may be traces. Cable 22, which has a multiplicity of signal pathways extending therethrough terminates in a connector 24, adapted to connect to an imaging station. Elements 16, which may be rubber bands, or some other form of elastic bands or clips, hel p to retain cable 22, to the side of endoscope 12. A tension member 30, such as a wire (which may also have some compressive strength) is attached to a bump 32 on ultrasound imaging head 20 a nd extends through a lumen 34 (FIG. 2) to emerge outside of a port 36 on the proximal end of endoscope 12, to be manipulated. I n embodiments, tension member 30, does not extend through lumen 34, but extends along the side of endoscope 12, and in embodiments is retained by elements 16, which are modified from the simple shapes shown in FIG. 1, to include eyelets, to create a guide path for tension member 30. In one embodiment, tension member 30 is connected to controls on the proximal end of endoscope 12, to facilitate manipulation. In embodiments, these controls may take the form of a spool, that can be easily let out, or drawn in. Endoscope 12 also is equipped with intrinsic controls for deflecting the tip of the insertion tube, to facilitate introduction to a site of interest.

In an alternative embodiment, tension member 30 is replaced by a tension member extending along the exterior of the endoscope, to a fixation point on the end of the endoscope. A physician may exert traction on tension member 30 in any one of a variety of ways, to cause ultrasound imaging head 20 to bend back toward retaining element 18, as permitted by a resiliently flexible neck 38 (FIG. 2). In one method a rotatable element is used to draw in tension member 30.

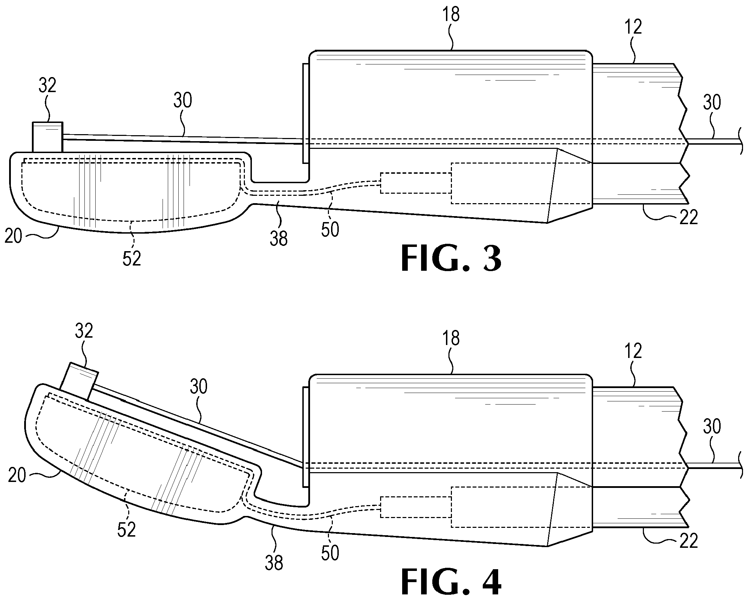

In preferred embodiment endoscope 12 includes an element at its distal end to guide the alignment of the retaining element 18. For example, endoscope 12 many include a groove at its distal end, into which a key element on retaining element 18 engages. In another embodiment, an orientation guide includes a peg that fits into the lumen 34 and is used to guide the correct orientation of retaining element 18. In one embodiment, assembly 14 is made for intended disposal, after a single use, and is used in this manner. In another embodiment, assembly 14 is constructed so as to be prepared and/or cleaned appropriately for reuse, after use, and then reused. Although until recently generally disinfection procedures were deemed adequate, the detection of instances of the spread of infection through endoscope has give rise to the use of high-end disinfection techniques for endoscopes. These disinfection techniques make use of chemicals to kill any pathogens left on the scope after use. Other disinfection or sterilization techniques may be used, including processing using of UV light and/or a gas, such as ozone. In the context of this application, the term "cleaning" encompasses all disinfection and sterilization techniques. Generally, the materials used in endoscopes are such that autoclaving an endoscope, or an attachment thereto is not feasible. Referring to FIGS. 3 and 4, a flex circuit 50, which passes through the flexible neck 38, electrically connects imaging head 20 to cable 22. Flex circuit 50 has an electrical lead for each transducer element in an ultrasound element array 52, resident in the ultrasound imaging head 20, to drive ultrasound element array 52 and relay signals from it. Array 52 is covered with a protective coating 53. In an alternative preferred embodiment, flex circuit 50 extends from imaging head 20 to (or through) connector 24, may define a plurality of coax cables and may directly contact the elements of the ultrasound array 52. In another alternative embodiment, cable 22 comprises a set of coax cable bound together with an adherent and protective substance, such as a polymer, and extends from connector 24 to imaging head 20. In yet another embodiment, a fiber optic cable is used in place of cable 22, with light to electric convertors at its distal end. I n any one of these arrangements elements 22 and 50 could be termed separately or in combination as a multiple signal pathway cable.

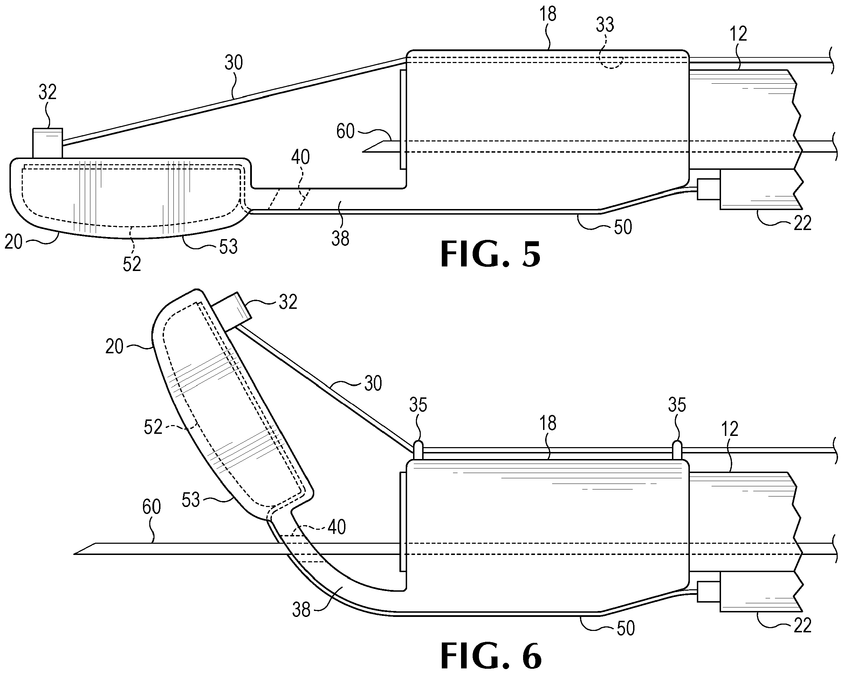

In a preferred embodiment a biopsy needle 60 (FIGS. 5 and 6), which forms the sharpened, distal portion of a long, flexible, hollow-core wire. This wire is sheathed in a flexible conduit (not shown), thin enough to extend through the lumen 34 and protecting endoscope 12 from being damaged by needle 60. Once the conduit reaches the distal end of endoscope 12, it may be pushed out to extend from lumen 34, and provide further guidance for needle 60, which is pushed out of the conduit at a point distal to the end of endoscope 12. Alternatively, the conduit may be pushed roughly to the end of lumen 34, with the needle pushed out of the conduit at that point. Referring to FIG. 5, in an alternative preferred embodiment tension member 30 extends through a channel 33 in retaining element 18 to reach bump 32. This figure also shows a needle 60 that has been pushed through a lumen of the endoscope 12 and is emerging from the distal end of the lumen. An aperture 40 is defined in neck 38, corresponding to an aperture in flex circuit 50, aligned with aperture 40. FIG. 6 shows an embodiment that is similar to that of FIG. 5, but instead with tension member 30 extending through a pair of eyelets 35, supported on the retaining element 18. As well as showing a slightly different embodiment, FIG. 6 also shows imaging head 20 retracted and needle 60 extending through aperture 40, as it would be in order to take a biopsy. Notably, in this position the needle would be within the field of view of ultrasound array 52. In embodiments, tension member 30 can pull head 20 into an obtuse angle, relative to the distal end of the endoscope 12. Generally, aperture 40 is in the shape of a long oval, so that the needle 60 can pass through it over a long range of degree of bending of neck 38. In another preferred embodiment, the flexible conduit is extended distally from lumen 34 into a v-shaped indentation (not shown) on surface of flexible neck 38, aligning the conduit so that the needle 60 is aligned to pass through aperture 40.

To use imaging assembly 10, ultrasound assembly 14 is attached to endoscope 12 by means of retaining element 18. In an alternative embodiment, rubber bands or clips 16 retain cable 22 to the side of endoscope 12. Imaging head 20 is then delivered to an area of interest, by means of standard endoscope introduction techniques. Imaging head 20 may then be moved to gain imagery of the area of interest by dedicated controls which control the ultrasound imaging head 20 deflection. If there appears to be a finding to be sampled, needle 60 may be introduced through an endoscope lumen and through aperture 40 and used to take a biopsy, inject a drug, or otherwise effect a medical procedure. Finally, needle 60 is retracted through the lumen of endoscope 12 and the endoscope is retrieved from the patient's body. In other embodiments, needle 60 is not included and an assembly that is similar to imaging assembly 10 but without needle 60 and related elements, is used for imaging alone.

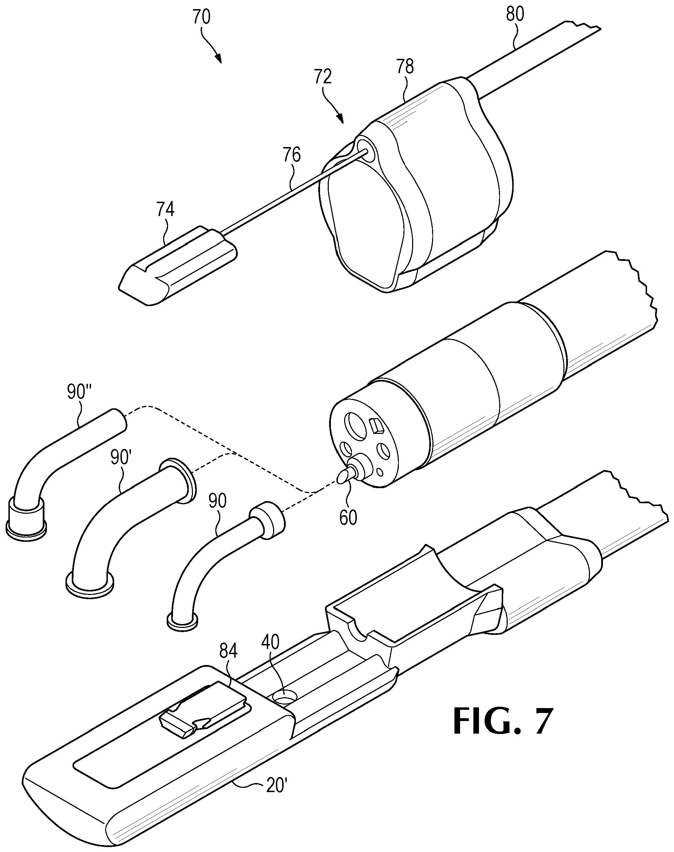

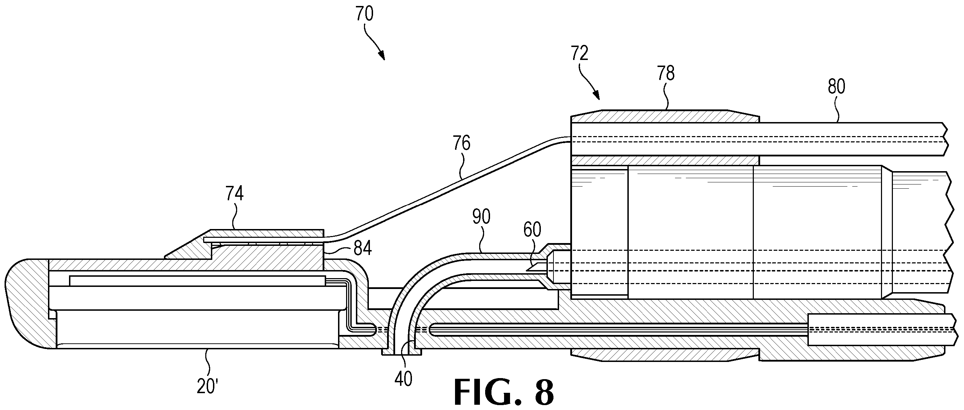

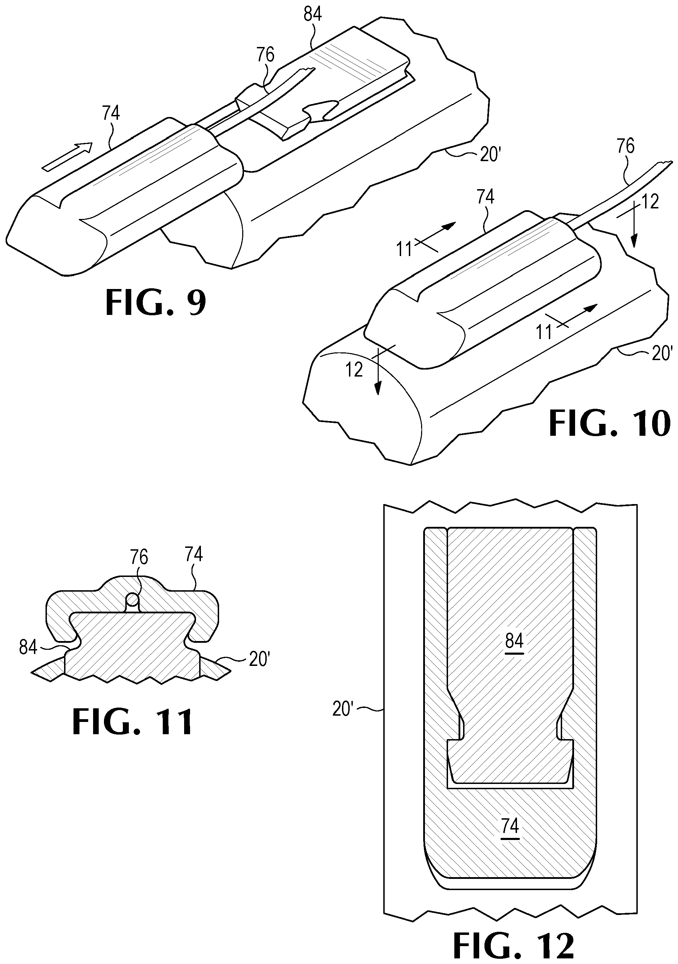

FIGS. 7-13 show an alternative embodiment 70 of the assembly 10, with the further innovation of a disposable head-movement sub-assembly 72, which includes a head clip 74, a movement cable 76, a cable clip 78 and a conduit 80, holding the major portion of movement cable 76. A clip-hold 84 is defined on the back of imaging head 20'. FIGS. 9-12 show engagement of head clip 74 to clip-hold 84. FIG. 9 shows head clip 74 distal to and being pulled back onto clip-hold 84, with FIG. 10 showing head clip 74 engaged to clip-hold 84 and FIGS. 11 and 12 showing different sectional views of head clip 74 and clip-hold 84 engaged together.

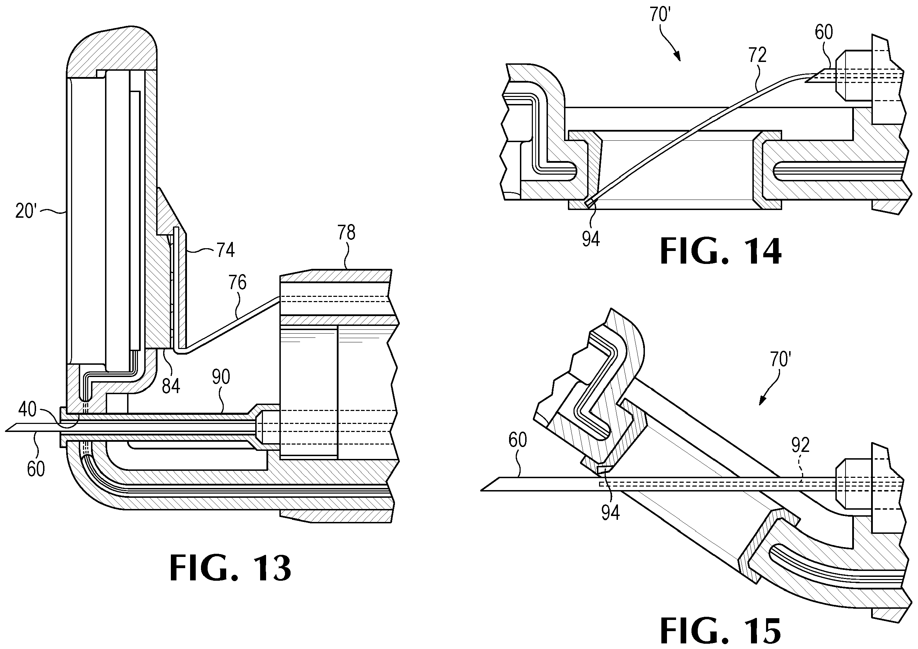

Another difference between assembly 70 and assembly 10 is the presence of a needle guide 90. FIG. 7 shows two additional variant needle guides 90' and 90". In assembly 10 it is possible that a needle 60 pushed out of a lumen of endoscope 12 could miss the aperture 40 in neck 38 and be blocked by neck 38 from further advancement. This might happen if a user attempted to push needle 60 into use when the neck 38 was not sufficiently pulled back, to bring aperture 40 into the correct position to let needle 60 pass through. The result could be damage caused to imaging head 20', cause by needle 60. A needle guide 90 engages with aperture 40, so that needle 60 will be guided to aperture 40 with certainty, or will be blocked by guide 90, when head 20' is not positioned correctly to align aperture 40 with the path of needle 60. FIG. 13 shows a needle guide 90 in use as head 20' is pulled fully back, to a forward-looking position as needle 60 is advanced through aperture 40, with the assistance of guide 90. It is a further advantage of assembly 70 (and assembly 10) that the head 20' can be moved to a forward-looking position as shown in FIG. 13, which is helpful to surgeons for some types of procedures. Referring to FIGS. 14 and 15, in a variant 70' to assembly 70, a needle guide 92 is provided in the form of a wire that needle 60 advances over. When not in use, needle guide 92 is retained in a needle-guide notch 94 (FIG. 15).

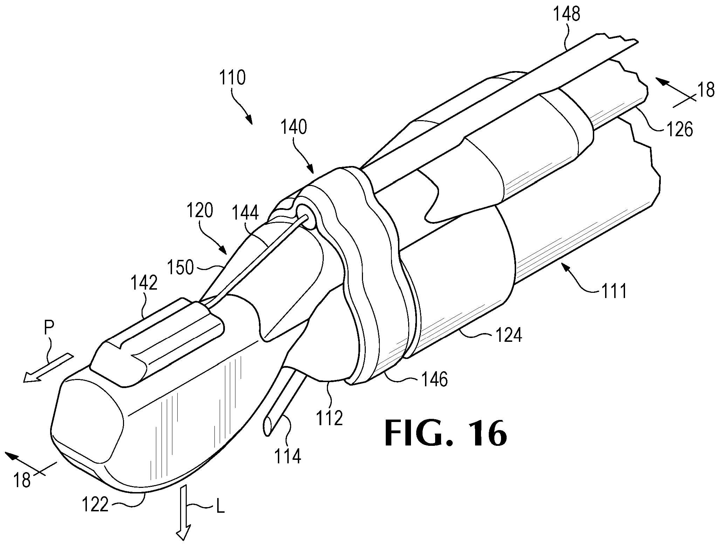

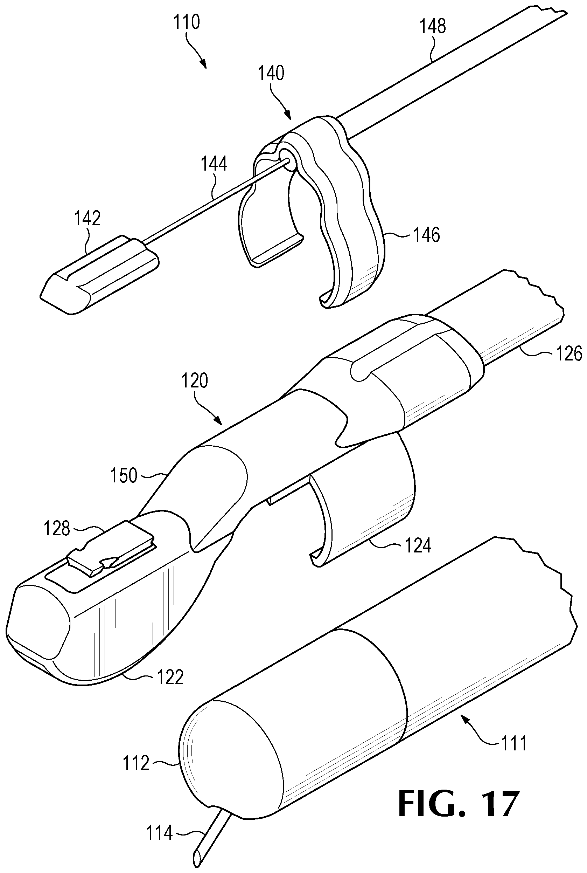

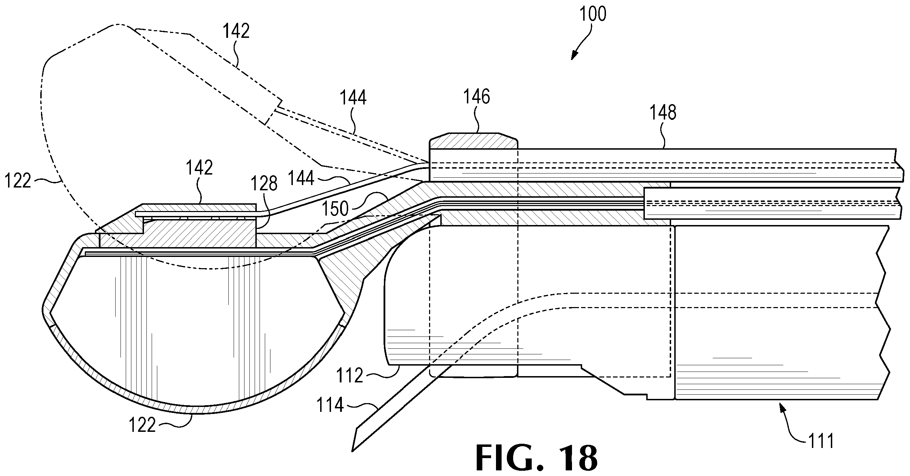

Referring now to FIGS. 16-18, a duodenoscope assembly 110 includes a duodenoscope 111 having a single-use needle guidance head 112 (shown most clearly in FIG. 18), having a needle guide 114 extending outwardly at an angle between a first lateral direction L and the distal direction P. Guidance head 112 can change the direction of needle guide 114, in response to varying user input via a tension member and a variable guide member (not shown). Referring to FIG. 16, a cable/head sub-assembly 120 includes an ultrasound imaging head 122, a scope clip 124, a multiple signal pathway cable 126, delivering signals to imaging head 122 and relaying signals from imaging head 122. The signal pathways of cable 126 may be electrical conductors, and more specifically may each be a coax cable or a trace on a flex circuit. Other forms of signal pathways are possible. Imaging head 122 is shown having a signal emission surface facing the first lateral direction L, and a clip-hold 128 (FIG. 17) is present on head 122 on a side displaced from said signal emission surface in a second lateral direction, opposed to said first lateral direction L. An imaging head movement sub-assembly 140 includes a head clip 142, shaped to engage to clip-hold 128, a movement cable 144 a cable clip 146 and a conduit 148, holding the major portion of movement cable 144. Referring to FIG. 11, when cable 144 is pulled it pulls back imaging head 122 as indicated by the dotted line. I n some embodiments sub-assembly 140 further includes an actuator (not shown) at the proximal end, to permit an operator to draw in cable 144, thereby pulling on imaging head 122 or let out cable 144, either pushing on imaging head 122 or permitting the resiliency of the material of cable 126 to place head 122 into a position more aligned with the longitudinal dimension of the duodenoscope 111, at its distal end. The actuator of cable 144 may take the form of a wheel, a lever or any other arrangement convenient to the user.

Because disinfection techniques typically require the application of chemicals in liquid form, thin crevices, into which liquid might not easily flow are generally undesirable. Accordingly, clip-hold 128 is designed so as not to define thin crevices with the imaging head 122. In alternative preferred embodiments, clip-hold 128 may have a shape that is similar to a knob, to further avoid defining any narrow crevices.

As noted in the background, the disinfection of devices such as assembly 110 is a matter of great concern, as there have been cases of the spread of strains of bacteria that are resistant to multiple antibiotics, by way of duodenoscope reuse. One area which may prove particularly difficult to sterilize is conduit 148, as movement cable 144 will tend to introduce body fluids into conduit 148 as cable 144 is pulled back into conduit 148, as imaging head 122 is moved back. To address this issue head movement sub-assembly is releasable and removeable from the remainder of assembly 110 and is made to be inexpensive enough to use a single time and then be disposed. This eliminates the possibility of infection being spread from patient to patient by way of sub-assembly 140. Cable/head sub-assembly 120 does not have a similar structure that would provide a hard-to-reach place, that would make disinfection difficult and will tend to be more expensive as it must contain a multiplicity of fine wires or other forms of signal pathways. Accordingly, cable/head sub-assembly 120 is designed to be cleaned and reused.

Before performing an endoscopic (duodenoscopic) surgery the surgeon would obtain an unused head movement sub-assembly 140 and attach it to the remainder of assembly 110. After use, the user detaches and disposes sub-assembly 140.

Referring to FIG. 16, movement cable 144 may be pulled back to cause head 122 to face in a more distal facing direction. Pushing cable 144 forward causes head 122 to adopt a lateral viewing angle as shown, in one embodiment due to resilience of neck 150, but in another due to stiffness and compressive strength in cable 144.

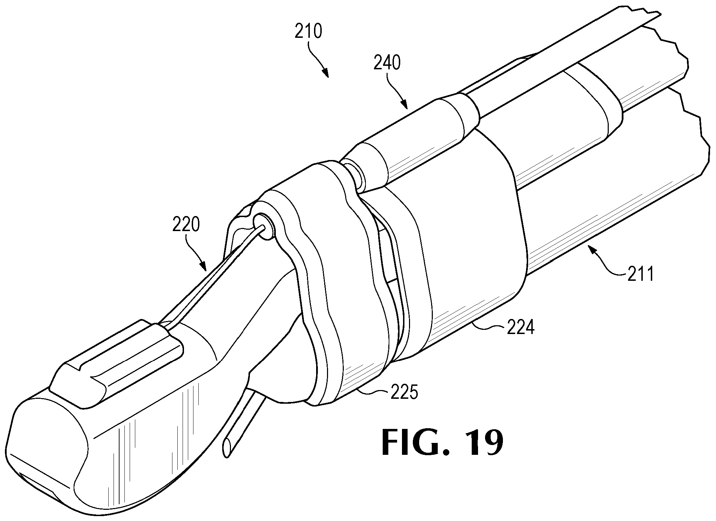

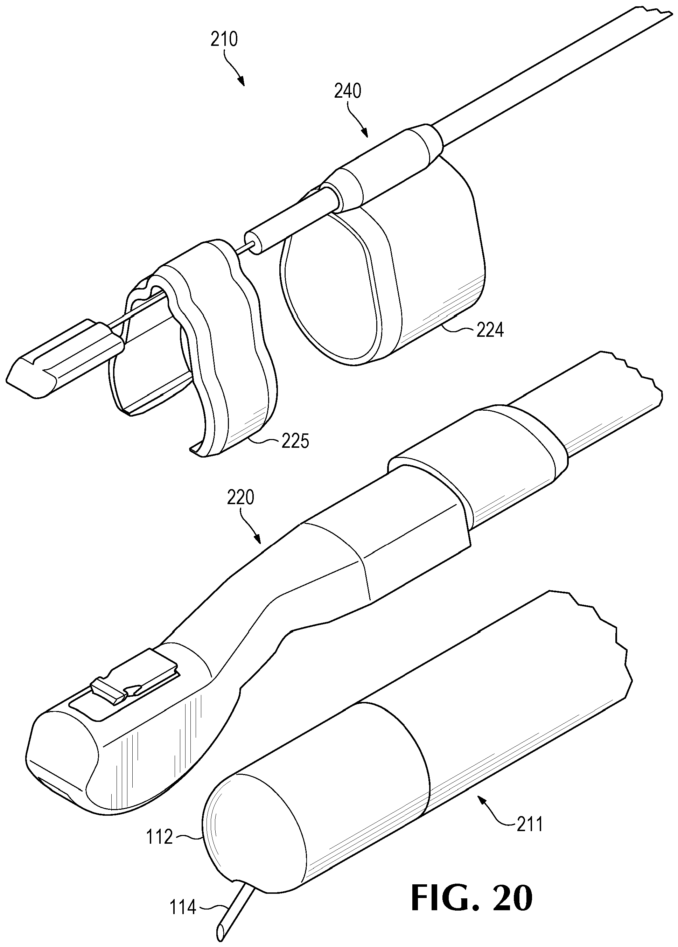

Referring to FIGS. 19 and 20, in an alternative preferred embodiment of a duodenoscope assembly 210, a holder 224 encompasses together both the duodenoscope 211, the cable/head sub-assembly 220 and the imaging head movement sub-assembly 240. A clip 225 also helps to hold the elements together.

### Industrial Applicability

The invention has industrial applicability in the field of manufacturing add-on devices for endoscopes.

While a number of exemplary aspects and embodiments have been discussed above, those possessed of skill in the art will recognize certain modifications, permutations, additions and sub-combinations thereof. It is therefore intended that the following appended claims and claims hereafter introduced are interpreted to include all such modifications, permutations, additions and sub-combinations as are within their true spirit and scope.
Embodiments of the invention are defined in the following clauses:
1. A method of adding elements to an endoscope, to create an ultrasound capable endoscope, comprising:
   a) providing an endoscope, having a distal end and an elongate body defining one or more lumens;
   b) providing an ultrasound assembly that includes:
      i) a multiple signal pathway connector,
      ii) a deformable neck, having a proximal end and a distal end;
      iii) an ultrasound transducer head supported by said distal end of said neck, and including an ultrasound transducer; and
      iv) a tension member, connected to said ultrasound transducer head, and extending to said proximal end of said endoscope;
      v) a set of signal pathways, extending from said ultrasound transducer to said multiconductor electrical connector; and
   c) attaching said ultrasound assembly to said endoscope, so that said ultrasound assembly extends along said elongate body of said endoscope, and so that said ultrasound transducer head is held by said neck at a position distal to said distal end of said endoscope, and arranging said tension member so that it extends from a proximal point of said endoscope to said transducer head;
   d) whereby said ultrasound transducer head can be introduced into a patient body cavity, with said endoscope, and may be angle adjusted by said tension member by pulling on said tension member to bend said neck thereby adjusting position of said ultrasound transducer head, and may communicate with an imaging station by means of said signal pathways, thereby permitting a user to more accurately aim the imaging head at a feature of interest; and
   e) further wherein said neck defines an aperture, permitting a needle to be extended from a lumen of said endoscope, through said aperture, to perform a medical procedure, when said neck is flexed by said tension member.
2. The method of clause 1, wherein said signal pathways are electrical conductors.
3. The method of clause 2, wherein said electrical conductors are coax cables.
4. The method of clause 2, wherein said electrical conductors are flex circuit traces.
5. The method of clause 2, wherein said ultrasound assembly further includes an attachment element, and said ultrasound assembly is attached to said endoscope, at least in part, by attaching said attachment element to said distal end of said endoscope.
6. The method of clause 5, wherein said attachment element is a clip sized and shaped to engage to said distal end of said endoscope.
7. The method of clause 1, wherein said neck is resiliently deformable.
8. The method of clause 1, wherein said aperture is an elongate oval, and wherein said needle can pass through said aperture when said neck is bent in any degree selected from a long range of degree of bending.
9. The method of clause 1, wherein when said neck is flexed by said tension member and said needle is passed through said aperture, said needle is visible in a field of view of said ultrasound transducer, thereby permitting a user to more accurately guide said needle.
10. An ultrasonic endoscope assembly, comprising:
   a) an endoscope defining one or more lumens, and a needle that can be pushed forward out of a said lumen, to collect a biopsy specimen;
   b) an ultrasound sub-assembly, attached to said endoscope and including:
      i) a communications cable including a set of conductors, having a distal end;
      ii) an ultrasound imaging head connected to said distal end of said cable;
   c) an imaging head movement sub-assembly, including a conduit that is releasably connected to said endoscope, said conduit containing a tension member that is releasably connected to said imaging head; and d) whereby said imaging head movement sub-assembly can be released from said endoscope and said imaging head, to be disposed of, after use.
11. The assembly of clause 10, wherein said endoscope is a duodenoscope, further including a needle guidance head that can be controllably used to adjust the angle at which a needle is advanced from said lumen to collect a specimen.
12. The assembly of clause 10, wherein said needle guidance head is removeable.
13. The assembly of clause 10, wherein said ultrasound sub-assembly is releasable from said endoscope.
14. The assembly of clause 10, wherein said distal end of said communications cable forms a neck that bends when said imaging head movement sub-assembly is used to pull said imaging head.
15. The assembly of clause 14, wherein said neck defines an aperture, to let said needle pass through said neck.
16. The assembly of clause 15, wherein said endoscope includes a needle guide that can be used to direct a needle out of said scope so as to pass through said aperture.
17. A method of processing endoscope assemblies, between use, wherein each endoscope assembly includes an endoscope, an ultrasound sub-assembly, including a communications cable connected to an ultrasound imaging head, and an imaging head movement sub-assembly: a) after surgical use, removing and disposing of said imaging head movement sub-assembly and removing and cleaning said ultrasound sub-assembly and cleaning said endoscope, resulting in a cleaned endoscope;
   b) before use, taking a cleaned ultrasound sub-assembly and placing it on said cleaned endoscope;
   c) opening a clean package containing a never-used imaging head movement sub-assembly and attaching it to said endoscope and ultrasound sub-assembly, thereby joining said endoscope, ultrasound sub-assembly and imaging head movement sub-assembly together.
18. The method of clause 17, wherein said cleaning said endoscope includes disposing of some portion of said endoscope and cleaning the remainder of said endoscope. 19. The method of clause 17, wherein said steps of cleaning include disinfection.
20. An endoscope add-on assembly adapted to be attached to a target endoscope, including:
   a) an ultrasound imaging sub-assembly, including a communications cable connected to an ultrasound imaging head;
   b) an imaging head movement sub-assembly, including a conduit, holding a tension member that is attached to said ultrasound imaging head;
   c) connective elements, adapted to permit said endoscope add-on assembly to be attached to said target endoscope;
   d) wherein said imaging head movement sub-assembly is detachable from said ultrasound imaging sub-assembly, thereby permitting said ultrasound imaging sub-assembly to be processed separately from said imaging head movement subassembly, after use.
21. The assembly of clause 20, wherein said target endoscope is an upper endoscope.
22. The assembly of clause 20, wherein said target endoscope is a duodenoscope.

## Claims

1. An imaging head movement sub-assembly configured to:
attach to said endoscope and ultrasound sub-assembly, thereby joining said endoscope, ultrasound sub-assembly and imaging head movement sub-assembly together, wherein the ultrasound sub-assembly includes an ultrasound imaging head and a communications cable connected to the ultrasound imaging head, and
be removed from the endoscope and the ultrasound sub-assembly, for disposal.

2. The imaging head movement sub-assembly of claim 1, wherein the imaging head movement sub-assembly is configured to attach to the endoscope when the ultrasound sub-assembly is placed on the endoscope.

3. An assembly comprising the imaging head sub-movement assembly of claim 1 or claim 2, further comprising the ultrasound sub-assembly.

4. The assembly of claim 3, wherein the ultrasound assembly includes a deformable neck, having a proximal end and a distal end,
wherein the ultrasound transducer head is supported by said distal end of said neck,
and
wherein the imaging head movement sub-assembly comprises a tension member, connected to said ultrasound transducer head, and extending to said proximal end of said endoscope, wherein applying force to said tension member causes said ultrasound transducer head to change position.

5. The assembly of claim 3, wherein the communications cable includes signal pathways that are electrical conductors.

6. The assembly of claim 5, wherein said electrical conductors are coax cables or
flex circuit traces.

7. The assembly of claim 3, wherein said ultrasound assembly further includes an attachment element, and said ultrasound assembly is attached to said endoscope, at least in part, by attaching said attachment element to a distal end of said endoscope.

8. The assembly of claim 7, wherein said attachment element is a clip sized and shaped to engage to said distal end of said endoscope.

9. The assembly of claim 4, wherein said neck is resiliently deformable.

10. The method of claim 4, wherein said neck defines an aperture, permitting a needle to be extended from a lumen of said endoscope, through said aperture, to perform a medical procedure, optionally wherein said aperture is an elongate oval, and wherein said needle can pass through said aperture when said neck is bent in any degree selected from a long range of degree of bending.

11. The method of claim 4, said neck defines an aperture, permitting a needle to be extended from a lumen of said endoscope, wherein when said needle is passed through said aperture, said needle is visible in a field of view of said ultrasound transducer, thereby permitting a user to more accurately guide said needle.

12. The assembly of claim 3, further comprising the endoscope, wherein said endoscope is a duodenoscope, further including a needle guidance head that can be controllably used to adjust the angle at which a needle is advanced from a lumen defined by the endoscope to collect a specimen.

13. The assembly of claim 4, wherein a distal end of said communications cable forms a neck that bends when said imaging head movement sub-assembly is used to pull said imaging head, optionally wherein said neck defines an aperture, to let said needle pass through said neck, optionally wherein said endoscope includes a needle guide that can be used to direct a needle out of said scope so as to pass through said aperture.

14. The assembly of claim 3, wherein the ultrasound sub-assembly is configured to be removed and cleaned after surgical use, for re-use and/or wherein the endoscope is a duodenoscope.

15. A clean package containing a never-used imaging head movement sub-assembly according to claim 1.
